# EUROPEAN PATENT APPLICATION

(11) **EP 4 837 571 A1**
(43) Date of publication of application: **11.11.2026**
(21) Application number: 25913929.3
(22) Date of filing: 02.04.2025
(51) Int. Cl.: A61K 31/37, A61K 36/484, A61P 17/14, A61Q 7/00, A61K 8/30, A61K 8/97

(54) **GLYCYCOUMARIN EXTRACT FOR PREVENTING HAIR LOSS OR PROMOTING HAIR GROWTH, COMPOSITION, AND USE**

(30) Priority: 13.03.2025 CN 202510297659
(71) Applicant: Novatide (Kunming) Biotech Co., Ltd, Kunming, Yunnan 650000 (CN)
(72) Inventor: CHENG, Yizhe, Kunming Yunnan 650000 (CN); GAO, Shaoyang, Kunming Yunnan 650000 (CN); LAI, Ren, Kunming Yunnan 650000 (CN); DING, Wenjiao, Kunming Yunnan 650000 (CN); QIN, Yulei, Kunming Yunnan 650000 (CN); ZHANG, Cheng, Kunming Yunnan 650000 (CN); XIA, Yu, Kunming Yunnan 650000 (CN)
(74) Representative: Lapienis, Juozas
(86) International application number: PCT/CN2025/086766
(87) International publication number: WO 2026/188605

(57) **Abstract**

The present invention discloses a glycycoumarin extract for preventing hair loss or promoting hair growth, and a composition and use thereof. The present invention specifically relates to a new use of glycycoumarin for the preparation of a product for preventing hair loss or promoting hair growth. The glycycoumarin is derived from a glycycoumarin extract extracted and purified from a raw material of liquorice. The present invention further relates to the glycycoumarin extract and a composition including the glycycoumarin extract. The present invention confirms for the first time that glycycoumarin can effectively prevent hair loss and promote the proliferation and differentiation of hair follicle cells to achieve the functions of preventing hair loss and promoting hair growth, with significant efficacy, no cytotoxicity, and high safety, filling the technical gap in the field of preventing hair loss and promoting hair growth using glycycoumarin. The glycycoumarin extract of the present invention can be industrially produced and prepared using a simple method with high efficiency and low and controllable cost, providing favorable conditions for the wide application of the glycycoumarin.

## Description

### BACKGROUND OF THE INVENTION

### 1. Technical Field

The present invention relates to the technical field of biomedicine, and in particular to a glycycoumarin extract for preventing hair loss or promoting hair growth, and a composition and use thereof.

### 2. Description of Related Art

Hair loss refers to a condition that hair falls out due to various reasons, and it includes both physiological and pathological hair loss. Normal physiological hair loss stems from the natural growth cycle of hair. It is normal to lose 50-100 hair per day, and new hair will grow gradually. Pathological hair loss, however, occurs as a result of complex causes, including genetic factors, imbalances in hormone levels, nutritional deficiencies, long-term mental stress and anxiety, unhealthy lifestyles, fungal or bacterial infection, head trauma surgery, or exposure to some chemical substances, etc., leading to congenital or acquired hair loss. Currently, the problem of hair loss is becoming increasingly severe. Data shows that there are over 250 million people with hair loss in China, with about 163 million men and 88 million women affected. Moreover, the group of people suffering from hair loss is getting younger, and the proportion of people experiencing hair loss before the age of 30 has significantly increased compared to the previous generation. More than 50% of patients provide feedback that hair loss has seriously affected their quality of life. Therefore, it is urgent to carry out research and development against the issue of hair loss.

At present, there are numerous methods for treating hair loss, each with its own advantages and disadvantages. In terms of drug therapy, finasteride (for men), spirolactone (for women) and other anti-androgenic drugs can be used to treat androgenetic alopecia. When combined with minoxidil tincture for topical and external use, these drugs can ameliorate the condition of hair loss. However, finasteride may have side effects such as hypaphrodisia, and needs to be taken for a long time. Once the medication is discontinued, hair loss may recur. During the initial use of minoxidil, hair loss may worsen, and some people may experience adverse reactions such as mild scalp itching or redness. For alopecia areata, hair growth can be promoted by the external use of minoxidil tincture, or by, if necessary, topic application of glucocorticoids such as halometasone ointment or oral administration of prednisone for hormonotherapy. However, long-term use of glucocorticoids may lead to atrophoderma, hypertrichosis, hormone dependent dermatitis, or other issues. In case of tinea capitis caused by fungal infection, it is necessary to take anti-fungal drugs such as terbinafine and ketoconazole, and disinfect relevant articles if necessary. However, the anti-fungal drugs may cause some damage to organs such as the liver, with a relatively long treatment cycle. Among surgical therapies, autogenous hair transplantation is an effective solution for treating severe and permanent hair loss, which can significantly improve the appearance, but with high surgery cost, risks such as infection and low hair follicle survival rate, and long postoperative recovery. In addition, a fractional laser therapy can be used to induce hair growth by promoting local blood circulation and angiogenesis. This therapy, however, requires multiple administration with short effect persistence, and a treatment course may be accompanied pain or other discomfort.

Liquorice is a perennial herb belonging to the genus Glycyrrhiza of the family Leguminosae. It is a traditional Chinese medicinal herb. The Chinese Pharmacopoeia lists three varieties as sources of medicinal licorice, including Glycyrrhiza uralensis Fisch., Glycyrrhiza inflata Bat., and Glycyrrhiza glabra L. Due to its homologous characteristics of medicine and food, liquorice has been classified as a traditional Chinese medicinal herb with both food and medicinal characteristics by the National Health Commission. Due to its therapeutic effects of tonifying the spleen and replenishing qi, clearing away heat and removing toxicity, expelling phlegm to arrest coughing, alleviating spasms and relieving pain, and coordinating the properties of herbs, liquorice is currently used to treat symptoms such as deficiency of the spleen and stomach, fatigue and lack of strength, palpitation and shortness of breath, cough with excessive phlegm, contracture pain in the stomach duct and abdomen as well as limbs, abscess and pus infection, and drug toxicity and harshness.

As can be seen from this, existing drugs for preventing hair loss obviously fail to meet the market demands and require urgent further improvement. How to develop a new glycycoumarin extract for preventing hair loss or promoting hair growth, and a composition and use thereof, for safe, reliable and effective addressing of hair loss issues, has become a target needing improvement in the current industry.

### BRIEF SUMMARY OF THE INVENTION

One of the technical problems to be solved by the present invention is to provide use of glycycoumarin for the preparation of a product for preventing hair loss or promoting hair growth.

Another technical problem to be solved by the present invention is to provide a glycycoumarin extract for preventing hair loss or promoting hair growth.

A further technical problem to be solved by the present invention is to provide a composition for preventing hair loss or promoting hair growth.

To achieve the objects above, the present invention provides the following technical solutions.

The present invention provides use of glycycoumarin for the preparation of a product for preventing hair loss or promoting hair growth.

Further, the glycycoumarin can promote vascular endothelial growth factor (VEGF) growth, increase an expression level of β-catenin gene, and reduce expression levels of Dickkopf-1 (DKK1), transforming growth factor-beta 2 (TGF-β2), and androgen receptor (AR) genes, to prevent androgenetic alopecia or promote hair growth.

Further, the product is a medicament or cosmetic, in which a content of the glycycoumarin is 0.01-2%, more preferably 0.2-1%.

Further, the glycycoumarin is derived from a glycycoumarin extract extracted and purified from a raw material of liquorice, and a purity of the glycycoumarin in the glycycoumarin extract is ≥10%.

The present invention further provides a glycycoumarin extract for preventing hair loss or promoting hair growth, wherein the glycycoumarin extract is extracted and purified from a raw material of liquorice, and a purity of glycycoumarin in the glycycoumarin extract is ≥10%.

Further, the glycycoumarin extract is extracted and purified using a method including:
Q1, selecting a raw material of liquorice, baking the liquorice to dryness, and slicing or crushing the liquorice;
Q2, adding the sliced or crushed liquorice to an extraction tank, and heating the extraction tank for reflux extraction using 5-20 mL/g ethyl acetate;
Q3, combining extracting solutions, concentrating the combined extracting solutions into an extractum, and redissolving the obtained extractum in ethyl acetate;
Q4, adding silica gel, with an amount accounting for 1/30-1/5 of an amount of the extractum, to a redissolved ethyl acetate solution from Q3, stirring a resulting mixture, letting the mixture stand for 30-120 min, collecting and concentrating a supernatant, and dissolving the concentrated supernatant in a mixed solvent of ethyl acetate and n-hexane, in which a content of the ethyl acetate is 30-60%; and
Q5, loading a resulting mixture from Q4 onto a silica gel column using a wet method, performing elution using an n-hexane-ethyl acetate system, during which a ratio of n-hexane to ethyl acetate is 100:0, 80:20, and 70:30 in sequence, collecting eluents in fractions, testing the collected eluents using a high-performance liquid chromatograph, combining the eluents containing glycycoumarin of different purities, and concentrating the combined eluent under reduced pressure into an extractum, thereby obtaining glycycoumarin extracts of varying purities.

Further, in Q1, the liquorice belongs to the varieties of liquorice grown in Korla, Xinjiang and Zhangye, Gansu, and liquorice slices have a thickness of 0.5-4 mm; in Q2, the extraction using the ethyl acetate is performed at a temperature of 60-90°C 2-3 times, each for 30-120 min; in Q3, an amount of the ethyl acetate used in redissolving is 6-15 mL/g; in Q4, the added silica gel is chromatographic silica gel of 300-400 meshes, and an amount of the mixed solvent of ethyl acetate and n-hexane is 5-10 mL/g; and in Q5, a loading volume for the silica gel column is 1/15-1/40 of an amount of the silica gel therein, wherein the n-hexane and the ethyl acetate at 100:0 allows for eluting with 3-4 column volumes, the n-hexane and the ethyl acetate at 80:20 allows for eluting with 5-6 column volumes, and the n-hexane and the ethyl acetate at 70:30 allows for eluting with 10-12 column volumes.

Further, in Q5, eluents containing glycycoumarin with a purity of ≥85% are combined and concentrated under reduced pressure into an extractum; the extractum is dissolved in one or more selected from petroleum ether, ethyl ether, methyl tert-butyl ether, n-pentane, ethyl acetate, n-hexane, cyclohexane, dichloromethane, and ethanol, and is then subjected to evaporative crystallization or cooling crystallization to obtain glycycoumarin crystals; and the glycycoumarin crystals are crushed to obtain glycycoumarin powder, in which the glycycoumarin has a purity of ≥98%.

The present invention further provides a composition for preventing hair loss or promoting hair growth, and the composition includes the glycycoumarin extract for preventing hair loss or promoting hair growth as described above.

Further, the composition is a medicament or cosmetic for topical and external use; a content of glycycoumarin in the composition is 0.01-2%, more preferably 0.2-1%; the composition further includes a physiologically acceptable excipient; and the composition is in a form of solution, ointment, hard ointment, gel, cream, lotion, liniment, tincture, emulsion, or patch.

With such a design, the present invention has at least the following advantages.
1. Based on experiments, the present invention demonstrates that glycycoumarin can promote VEGF production, increase the expression level of β-catenin gene, and reduce the expression levels of DKK1, TGF-β2, and AR genes, i.e., confirming for the first time that glycycoumarin can promote hair follicle angiogenesis, regulate the hair follicle growth cycle, promote the differentiation of follicle stem cells, enhance the survival and anti-apoptotic capacity of hair follicle cells, regulate the cytokine and growth factor networks around hair follicles, reduce the inhibition of Wnt/β-catenin signal pathway, reduce the apoptosis of hair follicle cells, decrease the inhibitory effect on the growth cycle of hair follicle cells, and inhibit the proliferation of sebaceous gland cells and the sebum secretion. In this way, the hair loss can be effectively prevented, and the proliferation and differentiation of hair follicle cells can be effectively promoted, thereby achieving the purpose of preventing hair loss and promoting hair growth. That is, the present invention provides a new use of glycycoumarin for preventing hair loss or promoting hair growth, with a clear mechanism of action, well-defined efficacy, no cytotoxicity, and high safety, filling the technical gap in the field of preventing hair loss and promoting hair growth using the glycycoumarin.
2. The glycycoumarin of the present invention is directly extracted and purified from the raw material of liquorice, and the glycycoumarin extracts of varying purity grades can be obtained according to needs by controlling the purification step. The present invention provides an industrial production process for the wide application of glycycoumarin using a simple method with high extraction efficiency and low and controllable cost, making it possible to provide favorable conditions for the further study and wide application of the glycycoumarin.

### BRIEF DESCRIPTION OF THE SEVERAL VIEWS OF THE DRAWINGS

Described above is only a summary of the technical solution of the present invention. For clearer understanding of the technical means of the present invention, the present invention will be further illustrated in detail below in conjunction with the accompanying drawings and the specific embodiments.
FIG. 1 shows the effect on VEGF levels in dermal papilla cells in individual groups in a dermal papilla cell anti-hair loss activity test according to Example 6 of the present invention.
FIG. 2 shows the effect on the expression of DKK1 gene in dermal papilla cells in individual groups in a dermal papilla cell anti-hair loss activity test according to Example 6 of the present invention.
FIG. 3 shows the effect on the expression of TGF-β2 gene in dermal papilla cells in individual groups in a dermal papilla cell anti-hair loss activity test according to Example 6 of the present invention.
FIG. 4 shows the effect on the expression of AR gene in dermal papilla cells in individual groups in a dermal papilla cell anti-hair loss activity test according to Example 6 of the present invention.
FIG. 5 shows the effect on the expression of β-catenin gene in dermal papilla cells in individual groups in a dermal papilla cell anti-hair loss activity test according to Example 6 of the present invention.
FIG. 6 shows the growth of hair on the backs of mice in individual groups after administration to the mice in individual groups for 12 days according to Example 7 of the present invention.

### DETAILED DESCRIPTION OF THE INVENTION

The technical solutions of the present invention will be illustrated in detail below in conjunction with specific examples. The examples below are only for the purpose of illustrating the present invention, and should not be regarded as limiting the scope of the present invention. The specific conditions not specified in the examples follow the conventional conditions or those recommended by the manufacturers. The reagents or instruments used without indicating manufacturers are all conventional products that are commercially available.

### Example 1

A glycycoumarin extract was prepared with a method including the steps as follows.
(1) Raw material pretreatment: liquorice from Zhangye, Gansu, was selected, baked to dryness, and cut into slices of 1 mm.
(2) Extraction: 5 kg of the treated liquorice was added to an extraction tank; 50 L of ethyl acetate was added; the extraction tank was heated for refluxing extraction 3 times at a temperature of 80°C; and extracting solutions were combined. Here, the first extraction was performed for 60 min, and the second and third extractions were performed for 75 min.
(3) Concentration: the extracting solution from (2) above was concentrated into an extractum, namely, the extractum of crude liquorice extract; and the extractum was redissolved in 6 mL/g ethyl acetate.
(4) Impurity removal: a silica gel with a weight being 1/20 of the weight of the extractum was added to the ethyl acetate solution from (3) above, where the silica gel was a chromatographic silica gel of 300-400 meshes; a resulting mixture was stirred and let stand for 60 min; a supernatant was collected and concentrated into an extractum; and the extractum was dissolved in a mixed solvent of ethyl acetate and n-hexane to obtain a column sample, where the mixed solvent was used at 8 mL/g, and the content of ethyl acetate in the mixed solvent of ethyl acetate and n-hexane was 40%.
(5) Silica gel column chromatography: the column sample from (4) above was loaded onto a silica gel column using a wet method, where the silica gel was a chromatographic silica gel of 300-400 meshes, a diameter/height ratio of the silica gel column was 1:8, a loading volume was 1/30 of the amount of silica gel; an n-hexane-ethyl acetate system was first used for eluting with 3-4 column volumes, where the volume fraction of the ethyl acetate in the n-hexane-ethyl acetate system was 0%; the n-hexane-ethyl acetate system was then used for eluting with 5-6 column volumes, where the volume fraction of the ethyl acetate in the n-hexane-ethyl acetate system was 20%; the n-hexane-ethyl acetate system was finally used for eluting with 10-12 column volumes, where the volume fraction of the ethyl acetate in the n-hexane-ethyl acetate system was 30%; and eluents were collected in fractions, with one bucket of eluent collected per column volume.

Meanwhile, glycycoumarin in each batch of eluent was tested using a high-performance liquid chromatograph; and the eluents with the content of glycycoumarin being ≥10% were combined and concentrated under reduced pressure to obtain a glycycoumarin extract with a purity of ≥20%.

Here, the chromatographic conditions for the high-performance liquid chromatograph were as follows: C18 chromatographic column, 4.6 mm × 250 mm, 5 µm; mobile phase A: methanol; mobile phase B: pure water; gradient elution with 70% A-90% A at a flow rate of 1 mL/min; column temperature 25°C; loading volume: 10 µL; and testing wavelength: 254 nm.

### Example 2

A glycycoumarin extract was prepared with a method including the steps as follows.
(1) Pretreatment of raw material: liquorice from Korla, Xinjiang, was selected, baked to dryness, and crushed by a crusher.
(2) Extraction: 10 kg of the treated liquorice was added to an extraction tank; 100 L of ethyl acetate was added; the extraction tank was heated for refluxing extraction 3 times at a temperature of 75°C; and extracting solutions were combined. Here, the first extraction was performed for 60 min, and the second and third extractions were performed for 75 min.
(3) Concentration: the extracting solution from (2) above was concentrated into an extractum, namely, the extractum of crude liquorice extract; and the extractum was redissolved in 10 mL/g ethyl acetate.
(4) Impurity removal: a silica gel with a weight being 1/10 of the weight of the extractum was added to the ethyl acetate solution from (3) above, where the silica gel was a chromatographic silica gel of 300-400 meshes; a resulting mixture was stirred and let stand for 60 min; a supernatant was collected and concentrated into an extractum; and the extractum was dissolved in a mixed solvent of ethyl acetate and n-hexane to obtain a column sample, where the mixed solvent was used at 8 mL/g, and the content of ethyl acetate in the mixed solvent of ethyl acetate and n-hexane was 40%.
(5) Separation through silica gel column: the column sample from (4) above was loaded onto a silica gel column using a wet method, where the silica gel was a chromatographic silica gel of 300-400 meshes, a diameter/height ratio of the silica gel column was 1:8, a loading volume was 1/25 of the amount of silica gel; an n-hexane-ethyl acetate system was first used for eluting with 3-4 column volumes, where the volume fraction of the ethyl acetate in the n-hexane-ethyl acetate system was 0%; the n-hexane-ethyl acetate system was then used for eluting with 5-6 column volumes, where the volume fraction of the ethyl acetate in the n-hexane-ethyl acetate system was 20%; the n-hexane-ethyl acetate system was finally used for eluting with 10-12 column volumes, where the volume fraction of the ethyl acetate in the n-hexane-ethyl acetate system was 30%; and eluents were collected in fractions, with one bucket of eluent collected per column volume.

Meanwhile, glycycoumarin in each batch of eluent was tested using a high-performance liquid chromatograph; the eluents with the content of glycycoumarin being ≥40% were combined and concentrated under reduced pressure to obtain a glycycoumarin extract with a purity of ≥60%.

### Example 3

A glycycoumarin extract was prepared with a method including the steps as follows.
(1) Pretreatment of raw material: liquorice from Zhangye, Gansu, was selected, baked to dryness, and cut into slices of 1 mm.
(2) Extraction: 10 kg of the treated liquorice was added to an extraction tank; 100 L of ethyl acetate was added; the extraction tank was heated for refluxing extraction 3 times at a temperature of 80°C; and extracting solutions were combined. Here, the first extraction was performed for 60 min, and the second and third extractions were performed for 75 min.
(3) Concentration: the extracting solution from (2) above was concentrated into an extractum, namely, the extractum of crude liquorice extract; and the extractum was redissolved in 10 mL/g ethyl acetate.
(4) Impurity removal: a silica gel with a weight being 1/20 of the weight of the extractum was added to the ethyl acetate solution from (3) above, where the silica gel was a chromatographic silica gel of 300-400 meshes; a resulting mixture was stirred and let stand for 90 min; a supernatant was collected and concentrated into an extractum; and the extractum was dissolved in a mixed solvent of ethyl acetate and n-hexane to obtain a column sample, where the mixed solvent was used at 6 mL/g, and the content of ethyl acetate in the mixed solvent of ethyl acetate and n-hexane was 50%.
(5) Separation through silica gel column: the column sample from (4) above was loaded onto a silica gel column using a wet method, where the silica gel was a chromatographic silica gel of 300-400 meshes, a diameter/height ratio of the silica gel column was 1:8, a loading volume was 1/20 of the amount of silica gel; an n-hexane-ethyl acetate system was first used for eluting with 3-4 column volumes, where the volume fraction of the ethyl acetate in the n-hexane-ethyl acetate system was 0%; the n-hexane-ethyl acetate system was then used for eluting with 5-6 column volumes, where the volume fraction of the ethyl acetate in the n-hexane-ethyl acetate system was 20%; the n-hexane-ethyl acetate system was finally used for eluting with 10-12 column volumes, where the volume fraction of the ethyl acetate in the n-hexane-ethyl acetate system was 30%; and eluents were collected in fractions, with one bucket of eluent collected per column volume.

Meanwhile, glycycoumarin in each batch of eluent was tested using a high-performance liquid chromatograph; and the eluents with the content of glycycoumarin being ≥60% were combined and concentrated under reduced pressure to obtain a glycycoumarin extract with a purity of ≥80%.

### Example 4

A glycycoumarin extract was prepared with a method including the steps as follows.
(1) Pretreatment of raw material: liquorice from Korla, Xinjiang, was selected, baked to dryness, and cut into slices of 1 mm.
(2) Extraction: 10 kg of the treated liquorice was added to an extraction tank; 100 L of ethyl acetate was added; the extraction tank was heated for refluxing extraction 3 times at a temperature of 80°C; and extracting solutions were combined. Here, the first extraction was performed for 60 min, and the second and third extractions were performed for 75 min.
(3) Concentration: the extracting solution from (2) above was concentrated into an extractum, namely, the extractum of crude liquorice extract; and the extractum was redissolved in 10 mL/g ethyl acetate.
(4) Impurity removal: a silica gel with a weight being 1/20 of the weight of the extractum was added to the ethyl acetate solution from (3) above, where the silica gel was a chromatographic silica gel of 300-400 meshes; a resulting mixture was stirred and let stand for 90 min; a supernatant was collected and concentrated into an extractum; and the extractum was dissolved in a mixed solvent of ethyl acetate and n-hexane to obtain a column sample, where the mixed solvent was used at 8 mL/g, and the content of ethyl acetate in the mixed solvent of ethyl acetate and n-hexane was 50%.
(5) Separation through silica gel column: the column sample from (4) above was loaded onto a silica gel column using a wet method, where the silica gel was a chromatographic silica gel of 300-400 meshes, a diameter/height ratio of the silica gel column was 1:8, a loading volume was 1/25 of the amount of silica gel; an n-hexane-ethyl acetate system was first used for eluting with 3-4 column volumes, where the volume fraction of the ethyl acetate in the n-hexane-ethyl acetate system was 0%; the n-hexane-ethyl acetate system was then used for eluting with 5-6 column volumes, where the volume fraction of the ethyl acetate in the n-hexane-ethyl acetate system was 20%; the n-hexane-ethyl acetate system was finally used for eluting with 10-12 column volumes, where the volume fraction of the ethyl acetate in the n-hexane-ethyl acetate system was 30%; and eluents were collected in fractions, with one bucket of eluent collected per column volume.

Meanwhile, glycycoumarin in each batch of eluent was tested using a high-performance liquid chromatograph; and the eluents with the content of glycycoumarin being ≥85% were combined and concentrated under reduced pressure to obtain a glycycoumarin extract with a purity of ≥95%.

Then, the glycycoumarin extract with a purity of ≥95% was dissolved in ethyl acetate and then subjected to evaporative crystallization to obtain a glycycoumarin extract with a purity of ≥98%; and the resulting glycycoumarin extract was dried in vacuum at low temperature and crushed to obtain glycycoumarin extract powder with a purity of ≥ 98%, named NWT-GU-P01.

### Example 5. Toxicity Test in Dermal Papilla Cells

### 1. Reagents and materials

MSCM culture medium (Gibco), PBS (Solarbio), MTT (Sigma), and DMSO (Sigma).

### 2. Instrumentation

CO₂ incubator (Thermo, 1501), superclean bench (Airtech, SW-CJ-1F), and microplate reader (BioTek, Epoch).

### 3. Cell strains

The cells used in this test were dermal papilla cells supplied by Guangdong BioCell Biotechnology Co. Ltd., with Batch No. 210727.

### 4. Test sample

Sample group: NWT-GU-P01, with test concentrations of 100 µg/mL, 50 µg/mL, 25 µg/mL, 12.5 µg/mL, 6.25 µg/mL, 3.125 µg/mL, 1.5625 µg/mL, and 0.78125 µg/mL.

Solvent control group: MSCM culture medium.

Positive control group: MSCM culture medium containing 10% DMSO.

Zeroing group: MSCM culture medium (cell-free).

### 5. Experimental method

Dermal papilla cells in a good condition in the exponential growth phase were seeded into a 96-well plate, and incubated overnight in a 5% CO₂ incubator at 37°C. Administration was performed when the cell plating rate in the 96-well plate reached 50-60%. In the solvent control group, 200 µL of the culture medium was added to each well; in the positive control group, 200 µL of the culture medium containing 10% DMSO was added to each well; in the sample group, 200 µL of the culture medium containing NWT-GU-P01 of corresponding concentrations was added; in the zeroing group, no cell was seeded, and only 200 µL of cell culture medium was added. After administration was completed, the 96-well plate was incubated in the 5% CO₂ incubator at 37°C for 24 hours. After the cells were incubated and cultured for 24 hours, a supernatant was discarded, and the MTT working solution (0.5 mg/mL) was added, and the plate was incubated at 37°C for 4 hours in the dark. After the incubation was completed, a supernatant was discarded, 150 µL of DMSO was added to each well, OD values were read at 490 nm, and the cell viability was calculated according to the following formula: $Relative cell viability \left(%\right) = \frac{\left(OD value of sample well-OD value of zeroing well\right) \times 100}{\left(OD value of solvent control group- OD value of zeroing group\right)} .$

### 6. Experimental results

MTT method is a method to test the viability and growth of cells, and the measured OD values were in direct proportion to the cell activity. Table 1 shows the testing results obtained by the MTT method in this example.

**Table 1. MTT testing results of glycycoumarin extract**

| Cell viability | Gradient concentration (µg/mL) | | | | | | | | Positive control | Solvent control |
|---|---|---|---|---|---|---|---|---|---|---|
| | 0.78125 | 1.5625 | 3.125 | 6.25 | 12.5 | 25 | 50 | 100 | 10% DMSO | / |
| Mean (%) | 98.56 | 98.78 | 98.44 | 91.05 | 71.45 | 33.91 | 0.46 | 0.80% | 0.89 | 100 |
| SD (%) | 0.46 | 4.19 | 1.04 | 0.82 | 1.79 | 1.27 | 0.07 | 0.07 | 0.00 | 3.52 |

As can be seen from Table 1, when the concentration of NWT-GU-P01 in the sample group is lower than 6.25 µg/mL, the viability of the dermal papilla cells is greater than 90%, indicating that the glycycoumarin NWT-GU-P01 does not show significant cell toxicity in the concentration range of 6.25 µg/mL based on the dermal papilla cells.

### Example 6. Anti-hair loss Activity Test in Dermal Papilla Cells

### 1. Reagents and materials

MSCM culture medium (Gibco), PBS (Solarbio), dihydrotestosterone (Sigma), minoxidil (Sigma), AG RNAex Pro Reagent (Accurate Biology), inverse transcription kit (Accurate Biology), fluorescent dye (Accurate Biology), and VEGF ELISA kit (Abcam).

### 2. Instrumentation

CO₂ incubator (Thermo, 150I), superclean bench (Airtech, SW-CJ-1F), microplate reader (BioTek, Epoch), standard PCR instrument (Bioer), and fluorescent quantitative PCR instrument (Roche, lightcycler 480 II).

### 3. Cell strains

The cells used in this test were dermal papilla cells supplied by Guangdong BioCell Biotechnology Co. Ltd., with Batch No. 210727.

### 4. Test sample

Sample group: NWT-GU-P01, with testing concentrations of 1.5 µg/mL, 3 µg/mL, and 6 µg/mL.

Blank control group: MSCM culture medium.

Negative control group: MSCM culture medium containing 800 nM dihydrotestosterone.

Positive control group: MSCM culture medium containing 800 nM dihydrotestosterone and 500 µM minoxidil.

### 5. Experimental method

Dermal papilla cells in a good condition in the exponential growth phase were seeded into a 6-well plate, and incubated overnight in a 5% CO₂ incubator at 37°C. Administration was performed when the cell plating rate in the 6-well plate reached 80-90%, with three replicate wells set in each group. In the blank control group, 2 mL of the culture medium was added to each well; in the negative control group, 2 mL of the culture medium containing 800 nM dihydrotestosterone was added to each well; in the positive control group, 2 mL of the culture medium containing 800 nM dihydrotestosterone and 500 µM minoxidil was added to each well; and in the sample group, 2 mL of the culture medium containing 800 nM dihydrotestosterone and NWT-GU-P01 of the corresponding concentration was added to each well. After administration was completed, the 6-well plate was incubated in the 5% CO₂ incubator at 37°C for 24 hours.

ELISA test: after the incubation was completed, the culture medium was collected in a centrifuge tube, after which a sample for ELISA testing was cryopreserved in a refrigerator at -80°C. Testing and analysis were then carried out according to the operating instructions of the ELISA kit.

Gene expression assay: after the incubation was completed, the previous medium was aspirated and discarded; the cells were washed twice with PBS; 1 mL of AG RNAex Pro Reagent was added to each well; and the cells were pipetted and disrupted, followed by the collection of samples. RNS was extracted, and reversely transcribed into cDNA for subsequent fluorescence quantitative PCR array, and the results were calculated using a 2-△△CT method.

Statistics and analysis of results: plotting was conducted using GraphPad Prism, with the results expressed as Mean ± SD. Comparisons between groups were subjected to statistics and analysis using t-test. Both statistics and analysis were based on the two-tailed test. P < 0.05 was considered a significant difference, and P < 0.01 was considered a highly significant difference.

### 6. Experimental results

Both dihydrotestosterone (DHT) and testosterone are bindable to androgen receptors (ARs), but the affinity of DHT is five times that of testosterone. The binding of DHT to AR leads to shortened hair growth cycle, gradually thinned hair in a growth phase, miniaturized hair follicles or even their atrophy, resulting in hair loss. This process mainly involves the Wnt signal pathway (i.e., the first signal of hair follicle development) and various cytokines (such as TGF-β and VEGF) involved in cell growth. The dermal papilla cells were stimulated using DHT, and changes in the levels of androgen receptor (AR), Wnt signal pathway (DKK1, β-catenin), TGF-β2 and VEGF were tested to evaluate the efficacy of the test sample against hair loss.

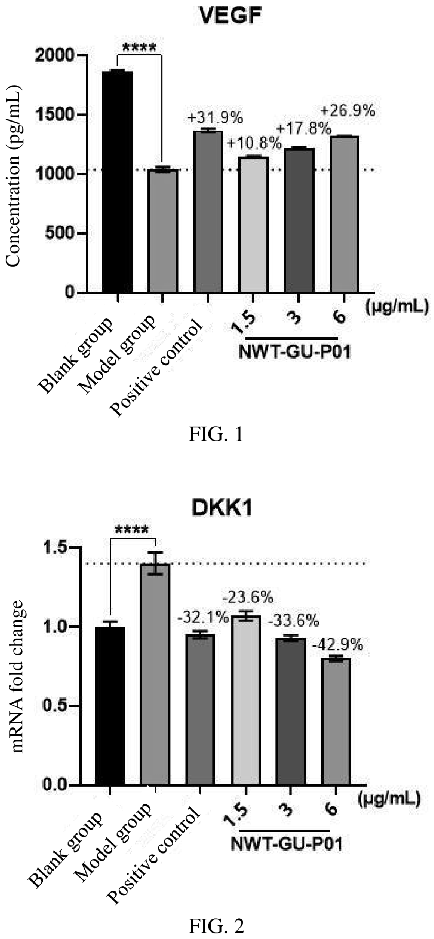

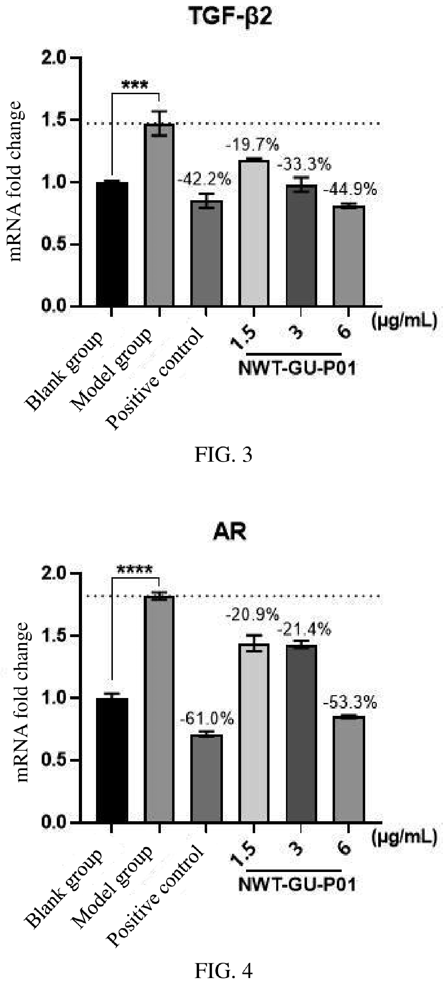

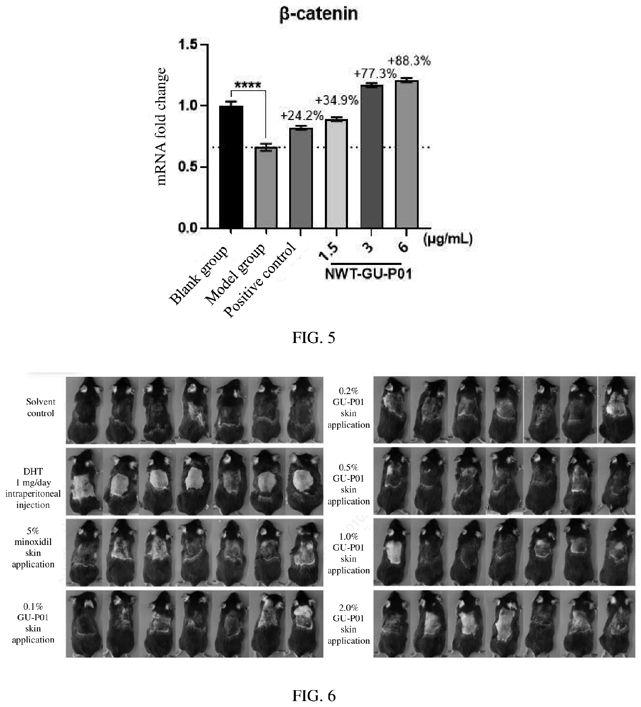

FIG. 1 shows the effect on VEGF levels in dermal papilla cells in individual groups in this example. FIG. 2 shows the effect on the expression of DKK1 gene in dermal papilla cells in individual groups in this example. FIG. 3 shows the effect on the expression of TGF-β2 gene in dermal papilla cells in individual groups in this example. FIG. 4 shows the effect on the expression of AR gene in dermal papilla cells in individual groups in this example. FIG. 5 shows the effect on the expression of β-catenin gene in dermal papilla cells in individual groups in this example.

### 7. Analysis of results

As can be seen from FIG. 1, the DHT module group significantly differs from the blank group, indicating successful modeling. At a concentration of 1.5 µg/mL, NWT-GU-P01 in the sample group has showed the ability to increase the VEGF levels, with an increase rate of 10.8%; and at concentrations of 3 µg/mL and 6 µg/mL, NWT-GU-P01 in the sample group shows a positive correlation with the increase in VEGF levels, with increase rates of 17.8% and 26.9%, respectively. NWT-GU-P01 at the concentration of 6 µg/mL in the sample group increases the VEGF levels to an extent close to that of the positive control group. This indicates that the glycycoumarin NWT-GU-P01 can significantly increase the VEGF levels.

As can be seen from FIG. 2, the DHT module group significantly differs from the blank group, indicating successful modeling. At a concentration of 1.5 µg/mL, NWT-GU-P01 in the sample group shows the ability to down-regulate the expression level of DKK1 genes, with a down-regulation rate of 23.6%; and at concentrations of 3 µg/mL and 6 µg/mL, NWT-GU-P01 in the sample group shows a positive correlation with the down-regulation in the expression levels of DKK1 genes, with down-regulation rates of 33.6% and 42.9%, respectively, both of which are more than that of the positive control group. This indicates that the glycycoumarin NWT-GU-P01 can significantly decrease the expression level of DKK1 genes.

As can be seen from FIG. 3, the DHT module group significantly differs from the blank group, indicating successful modeling. At a concentration of 1.5 µg/mL, NWT-GU-P01 in the sample group shows the ability to down-regulate the expression level of TGF-β2 genes, with a down-regulation rate of 19.7%; and at concentrations of 3 µg/mL and 6 µg/mL, NWT-GU-P01 in the sample group shows a positive correlation with the down-regulation in the expression levels of TGF-β2 genes, with down-regulation rates of 33.3% and 44.9%, respectively. The down-regulation rate of the NWT-GU-P01 at the concentration of 6 µg/mL in the sample group is also more than that of the positive control group. This indicates that the glycycoumarin NWT-GU-P01 can significantly decrease the expression level of TGF-β2 genes.

As can be seen from FIG. 4, the DHT module group significantly differs from the blank group, indicating successful modeling. At a concentration of 1.5 µg/mL, NWT-GU-P01 in the sample group shows the ability to down-regulate the expression level of AR genes, with a down-regulation rate of 20.9%; and at concentrations of 3 µg/mL and 6 µg/mL, NWT-GU-P01 in the sample group shows a positive correlation with the down-regulation in the expression levels of AR genes, with down-regulation rates of 21.4% and 53.3%, respectively. The down-regulation rate of the NWT-GU-P01 at the concentration of 6 µg/mL in the sample group is close to that of the positive control group. This indicates that the glycycoumarin NWT-GU-P01 can significantly decrease the expression level of AR genes.

As can be seen from FIG. 5, the DHT module group significantly differs from the blank group, indicating successful modeling. At a concentration of 1.5 µg/mL, NWT-GU-P01 in the sample group shows the ability to up-regulate the expression level of β-catenin gene, with an increase rate of 34.9%; and at concentrations of 3 µg/mL and 6 µg/mL, NWT-GU-P01 in the sample group shows a positive correlation with the up-regulation in the expression levels of β-catenin genes, with up-regulation rates of 77.3% and 88.3%, respectively, both of which are more than that of the positive control group. This indicates that the glycycoumarin NWT-GU-P01 can significantly up-regulate the expression level of β-catenin gene.

In summary, the glycycoumarin NWT-GU-P01 has the effects of increasing the VEGF levels, down-regulating the expression levels of DKK1, TGF-β2 and AR genes, and up-regulating the expression level of β-catenin gene, demonstrating a significant effect of preventing hair loss.

### Example 7. Activity Test in Mice with DHT-induced Hair Loss

### 1. Reagents and materials

Dihydrotestosterone (DHT), minoxidil, corn oil, rosin, beeswax, isoflurane, and maintenance feed for SPF rats and mice (Beijing Keao Xieli Feed Co., Ltd.).

### 2. Experimental animals

SPF-grade male C57BL/6J mice of six weeks old were purchased from SPF (Beijing) Biotechnology Co., Ltd. The mice were raised in a barrier environment with alternating light and dark conditions for 12 hours, with free access to food and water.

### 3. Experimental groups

1) Blank control group: the hair was removed, and skin application of a solvent of NWT-GU-P01 was conducted.
2) Modeling group: the hair was removed, and intraperitoneal injection of DHT (1 mg/day) and skin application of a solvent of NWT-GU-P01 were conducted.
3) Positive control group: the hair was removed, and intraperitoneal injection of DHT (1 mg/day) and skin application of 5% minoxidil were conducted.
4) 0.1% NWT-GU-P01 administration group: the hair was removed, and intraperitoneal injection of DHT (1 mg/day) and skin application of 0.1% NWT-GU-P01 were conducted.
5) 0.2% NWT-GU-P01 administration group: the hair was removed, and intraperitoneal injection of DHT (1 mg/day) and skin application of 0.2% NWT-GU-P01 were conducted.
6) 0.5% NWT-GU-P01 administration group: the hair was removed, and intraperitoneal injection of DHT (1 mg/day) and skin application of 0.5% NWT-GU-P01 were conducted.
7) 1% NWT-GU-P01 administration group: the hair was removed, and intraperitoneal injection of DHT (1 mg/day) and skin application of 1% NWT-GU-P01 were conducted.
8) 2% NWT-GU-P01 administration group: the hair was removed, and intraperitoneal injection of DHT (1 mg/day) and skin application of 2% NWT-GU-P01 were conducted.

### 4. Experimental method

The mice were adaptively fed for 7 days, and then randomly divided into 8 groups, with 7 mice in each group. The mice were anesthetized with isoflurane; after their back skin was shaved, a mixture of rosin and wax that had been and mixed melted at a ratio of 1:1 and stored at 50°C was evenly applied to the backs of the mice; after the mixture was cooled, the hair of the mice was removed; and after no significant abnormal reaction was observed in the skin of the mice, modeling was started.

DHT was dissolved in the corn oil to prepare a 10 mg/mL DHT solution, and except for the blank group, the mice in other groups were intraperitoneally injected with 0.1 mL (1 mg) of DHT in the morning to establish androgenetic alopecia models. Based on the experimental group, a solvent, 5% minoxidil, or NWT-GU-P01 of a corresponding concentration was applied to the hair removal area. Administration was performed once a day, for 17 consecutive days.

Starting from the date of administration, the back conditions of the mice were photographed once on Days 0, 3, 6, 9, 12, 15, and 17.

### 5. Experimental results

Androgenetic alopecia is a symptom of hair thinning and loss caused by the effects of testosterone on hair follicles. DHT-induced androgenetic alopecia in mice is a common preparation method, in which DHT is injected to simulate the physiological process of human androgenetic alopecia, and a test substance is administered for treatment, so as to evaluate the effect of the test substance on preventing hair loss and promoting hair growth.

FIG. 6 shows the growth of hair on the backs of mice in individual groups after administration to the mice in individual groups for 12 days in this example.

### 6. Analysis of results

As can be seen from FIG. 6, compared with the solvent control group, the DHT modeling group shows significantly reduced hair growth, indicating successful modelling. Compared with the DHT modeling group, the minoxidil (positive control) group shows significantly restored hair growth. Compared with the DHT modeling group, the NWT-GU-P01 administration group shows the mouse hair growth restored to different degrees at the test concentrations of 0.1%, 0.2%, 0.5%, 1%, and 2%, and shows dose correlation within the concentration range of 0.1% to 0.5%, where the restoration level of mouse hair growth at the test concentration of 1% is equivalent to or slightly lower than that at the test concentration of 0.5%. At the high concentration of 2%, the restoration level of mouse hair growth decreases. This indicates that the optimal effective concentration of the glycycoumarin NWT-GU-P01 is 0.2-1%.

In summary, in the DHT-induced mouse hair loss model, the glycycoumarin NWT-GU-P01 exhibits a significant effect of promoting hair regeneration, with the optimal effective concentration of 0.2-1%.

The above description only provides preferred embodiments of the present invention, and is not intended to limit the present invention in any form. Some simple alternations, equivalent variations or modifications made by those skilled in the art by using the technical content disclosed above shall fall within the protection scope of the present invention.

## Claims

1. Use of glycycoumarin for the preparation of a product for preventing hair loss or promoting hair growth.

2. The use according to claim 1, wherein the glycycoumarin can promote vascular endothelial growth factor VEGF growth, increase an expression level of β-catenin gene, and reduce expression levels of Dickkopf-1 DKK1, transforming growth factor-beta 2 TGF-β2, and androgen receptor AR genes, to prevent androgenetic alopecia or promote hair growth.

3. The use according to claim 2, wherein the product is a medicament or cosmetic, in which a content of the glycycoumarin is 0.01-2%.

4. The use according to claim 1, wherein the glycycoumarin is derived from a glycycoumarin extract extracted and purified from a raw material of liquorice, and a purity of the glycycoumarin in the glycycoumarin extract is ≥10%.

5. A glycycoumarin extract for preventing hair loss or promoting hair growth, wherein the glycycoumarin extract is extracted and purified from a raw material of liquorice, and a purity of glycycoumarin in the glycycoumarin extract is ≥10%.

6. The glycycoumarin extract for preventing hair loss or promoting hair growth according to claim 5, wherein the glycycoumarin extract is extracted and purified by a method comprising:
Q1, selecting a raw material of liquorice, baking the liquorice to dryness, and slicing or crushing the liquorice;
Q2, adding the sliced or crushed liquorice to an extraction tank, and heating the extraction tank for reflux extraction using 5-20 mL/g ethyl acetate;
Q3, combining extracting solutions, concentrating the combined extracting solutions into an extractum, and redissolving the obtained extractum in ethyl acetate;
Q4, adding silica gel, with an amount accounting for 1/30-1/5 of an amount of the extractum, to a redissolved ethyl acetate solution from Q3, stirring a resulting mixture, letting the mixture stand for 30-120 min, collecting and concentrating a supernatant, and dissolving the concentrated supernatant in a mixed solvent of ethyl acetate and n-hexane, in which a content of the ethyl acetate is 30-60%; and
Q5, loading a resulting mixture from Q4 onto a silica gel column using a wet method, performing elution using an n-hexane-ethyl acetate system, during which a ratio of n-hexane to ethyl acetate is 100:0, 80:20, and 70:30 in sequence, collecting eluents in fractions, testing the collected eluents using a high-performance liquid chromatograph, combining the eluents containing glycycoumarin of different purities, and concentrating the combined eluent under reduced pressure into an extractum, thereby obtaining glycycoumarin extracts of varying purities.

7. The glycycoumarin extract for preventing hair loss or promoting hair growth according to claim 6, wherein in Q1, liquorice slices have a thickness of 0.5-4 mm; in Q2, the extraction using the ethyl acetate is performed at a temperature of 60-90°C 2-3 times, each for 30-120 min; in Q3, an amount of the ethyl acetate used in redissolving is 6-15 mL/g; in Q4, the added silica gel is chromatographic silica gel of 300-400 meshes, and an amount of the mixed solvent of ethyl acetate and n-hexane is 5-10 mL/g; and in Q5, a loading volume for the silica gel column is 1/15-1/40 of an amount of the silica gel therein, wherein the n-hexane and the ethyl acetate at 100:0 allows for eluting with 3-4 column volumes, the n-hexane and the ethyl acetate at 80:20 allows for eluting with 5-6 column volumes, and the n-hexane and the ethyl acetate at 70:30 allows for eluting with 10-12 column volumes.

8. The glycycoumarin extract for preventing hair loss or promoting hair growth according to claim 7, wherein in Q5, eluents containing glycycoumarin with a purity of ≥85% are combined and concentrated under reduced pressure into an extractum; the extractum is dissolved in one or more selected from petroleum ether, ethyl ether, methyl tert-butyl ether, n-pentane, ethyl acetate, n-hexane, cyclohexane, dichloromethane, and ethanol, and is then subjected to evaporative crystallization or cooling crystallization to obtain glycycoumarin crystals; and the glycycoumarin crystals are crushed to obtain glycycoumarin powder, in which the glycycoumarin has a purity of ≥98%.

9. A composition for preventing hair loss or promoting hair growth, comprising the glycycoumarin extract for preventing hair loss or promoting hair growth according to any one of claims 5 to 8.

10. The composition for preventing hair loss or promoting hair growth according to claim 9, wherein the composition is a medicament or cosmetic for topical and external use; a content of glycycoumarin in the composition is 0.01-2%; the composition further comprises a physiologically acceptable excipient; and the composition is in a form of solution, ointment, hard ointment, gel, cream, lotion, liniment, tincture, emulsion, or patch.
